# EUROPEAN PATENT SPECIFICATION

(11) **EP 4 316 369 B1**
(45) Date of publication and mention of the grant of the patent: **23.09.2026**
(21) Application number: 22774146.9
(22) Date of filing: 18.03.2022
(51) Int. Cl.: A61B 5/145

(54) **REAL-TIME BLOOD GLUCOSE MONITORING APPARATUS AND MANUFACTURING METHOD THEREFOR**
ECHTZEIT-BLUTZUCKERÜBERWACHUNGSVORRICHTUNG UND HERSTELLUNGSVERFAHREN DAFÜR
APPAREIL DE SURVEILLANCE DE LA GLYCÉMIE EN TEMPS RÉEL ET SON PROCÉDÉ DE FABRICATION

(30) Priority: 23.03.2021 CN 202110307087
(43) Date of publication of application: 07.02.2024
(73) Proprietor: MicroTech Medical (Hangzhou) Co., Ltd., Hangzhou, Zhejiang 311121 (CN)
(72) Inventor: ZHENG, Pan, Hangzhou, Zhejiang 311121 (CN); WANG, Guodong, Hangzhou, Zhejiang 311121 (CN); SONG, Zhe, Hangzhou, Zhejiang 311121 (CN); YU, Fei, Hangzhou, Zhejiang 311121 (CN)
(74) Representative: ZBM Patents - Zea, Barlocci & Markvardsen
(86) International application number: PCT/CN2022/081702
(87) International publication number: WO 2022/199485

(56) References cited:
- CN-A- 112 120 709
- CN-U- 210 056 040
- CN-U- 215 078 572
- KR-A- 20200 131 380
- US-A1- 2019 336 054

## Description

### TECHNICAL FIELD

The present disclosure relates to the technical field of blood glucose monitoring, and in particular to a split-type real-time blood glucose monitoring apparatus and a manufacturing method therefor.

### BACKGROUND

The implementation of real-time blood glucose monitoring can better control the blood glucose changes of diabetic patients. It has important guiding significance for life rules, activities, exercise, diet and rational drug use, and can help patients find problems at any time and go to the hospital in time.

A real-time blood glucose monitoring apparatus comprises a sensor assembly and an emitter assembly. The sensor assembly and the emitter assembly have a split structure; wherein the sensor assembly comprises elements such as a blood glucose sensor, a monitoring needle of which is implanted subcutaneously for detecting blood glucose levels; and the emitter assembly comprises a circuit board. When the monitoring apparatus is in use, the sensor assembly is attached to the human skin, and the emitter assembly is regularly connected to the sensor assembly so as to cause the circuit in the blood glucose monitoring apparatus to form a closed-loop circuit, so that the blood glucose content data detected by the blood glucose sensor is sent to a terminal device via the emitter assembly to perform operations for doctors or users such as viewing and recording by using the terminal device.

Since the sensor assembly needs to be attached to the human skin during use, in order to reduce the influence brought by the attached sensor assembly, it is necessary to miniaturize the sensor assembly. In the existing real-time blood glucose monitoring apparatuses, in order to transmit the signals from sensors to circuit boards, nearby contact conduction is required, thus limiting the layout form or limiting the miniaturization of the volume.

US 2019/336054 A1 discloses a blood glucose monitoring apparatus according to the preamble of claim 1.

### SUMMARY

The object of the present invention is achieved with a real-time blood glucose monitoring apparatus according to claim 1, and a method for manufacturing such an apparatus according to claim 7.

In view of this, the present disclosure provides a real-time blood glucose monitoring apparatus and a manufacturing method therefor. In the apparatus, a conductive plating is used as a circuit board in a sensor assembly, and a protruding connecting structure is used as a connecting contact. This setting can optimize the structure of the blood glucose monitoring apparatus, and is beneficial to the miniaturization of product. One aspect of the present disclosure provides a real-time blood glucose monitoring apparatus, comprising a sensor assembly and an emitter assembly, wherein the sensor assembly comprises a first shell, and a conductive plating, a blood glucose sensor and a battery which are arranged on the first shell, and the emitter assembly comprises a second shell and a circuit board in the second shell. The conductive plating is arranged on a shell wall of the first shell, and is connected with the blood glucose sensor and the battery. The conductive plating has at least one first connecting structure. The circuit board is provided with at least one second connecting structure. The sensor assembly and the emitter assembly are clamped so that the first connecting structure and the second connecting structure are connected, and the conductive plating and the circuit board form a closed-loop monitoring circuit.

Optionally, the first connecting structure is a protruding column, on a wall of which is provided a plurality of to-be-connected terminals of the conductive plating. The second connecting structure is a connector, a first end of which is connected to the circuit board and a second end of which is provided with an inwardly concave plug-in recess. On an inner wall of the plug-in recess are provided reeds connected with the circuit board. When the first connecting structure and the second connecting structure are connected, the protruding column is plugged into the plug-in recess, and the reeds abut against the to-be-connected terminals of the conductive plating.

Optionally, the plurality of to-be-connected terminals of the conductive plating are distributed along the wall of the protruding column.

Optionally, the conductive plating has a first connecting structure, and the circuit board is provided with a second connecting structure.

Optionally, the first shell comprises a first upper shell and a first lower shell, the edges of which are connected; the conductive plating is arranged on a surface of the first lower shell, and the protruding column is fixedly connected to the surface of the first lower shell.

Optionally, the second shell comprises a second upper shell and a second lower shell, the edges of which are connected.

Optionally, the first upper shell is provided with a first through-hole, an edge of which is provided with a first sleeve. The protruding column is arranged within and runs through the first sleeve. A first annular insertion groove is formed between an outer wall of the first sleeve and the first upper shell. The center lines of the protruding column, the first sleeve and the first annular insertion groove coincide. The second lower shell is provided with a second through-hole, an edge of which is provided with a second sleeve protruding from the outer surface of the second lower shell. The connector is arranged within and runs through the second sleeve, andthere is a defined spacing between an outer wall of the connector and an inner wall of the second sleeve, thereby forming a second annular insertion groove. The center lines of the connector, the second sleeve and the second annular insertion groove coincide. When the first connecting structure and the second connecting structure are connected, the first sleeve is inserted into the second annular insertion groove, and the second sleeve is inserted into the first annular insertion groove.

Optionally, a sealing structure for blocking a gap between the first sleeve and the second sleeve is provided between the outer wall of the first sleeve and the inner wall of the second sleeve.

Optionally, the sealing structure comprises at least one sealing ring, which is sleeved on the outer wall of the first sleeve.

Optionally, an outer surface of the first lower shell is provided with medical adhesive tape, and the blood glucose sensor is arranged in a middle portion of the inner surface of the first lower shell, with a detecting end of the blood glucose sensor penetrating out of the first lower shell.

Another aspect of the present disclosure also provides a method for manufacturing a real-time blood glucose monitoring apparatus, comprising: plating a conductive plating on a shell of a sensor assembly, and connecting a battery and a blood glucose sensor to the conductive plating to form a circuit structure in the sensor assembly; providing a first connecting structure connected to the circuit structure, providing a second connecting structure connected to a circuit board of an emitter assembly, and communicating the first connecting structure with the second connecting structure so that the circuit structure and the circuit board are connected to form a closed-loop monitoring circuit.

Optionally, the step of providing a first connecting structure connected to the circuit structure comprises: incorporating a protruding column onto the shell of the sensor assembly or fabricating a protruding column on the shell of the sensor assembly; arranging a plurality of to-be-connected terminals of the conductive plating on side walls of the protruding column; the step of providing a second connecting structure connected with a circuit board of an emitter assembly comprises: providing a connector with a plug-in recess on the circuit board, and providing reeds having the same number as the terminals of the conductive plating on an inner wall of the plug-in recess, the reeds being connected to the circuit board; by plugging the protruding column into the plug-in recess, the to-be-connected terminals of the conductive plating abut against and communicates with the reeds, and then the circuit structure and the circuit board are connected to form a closed-loop monitoring circuit.

Optionally, the conductive plating is plated on the shell of the sensor assembly by molded interconnection device process and/or laser direct structuring process.

According to the technical solution of the present disclosure, a conductive plating is arranged on the shell of the sensor assembly, and the conductive plating replaces the existing circuit board structures. By reducing the circuit board structures, the sensor assembly can be further miniaturized. Furthermore, the use of the conductive plating allows for the centralization of the contacts for connection, thereby reducing distribution points of the contacts for connection on the sensor assembly and the emitter assembly, and reducing the space occupied by a contact structure, thereby facilitating the miniaturization of the product.

### BRIEF DESCRIPTION OF THE DRAWINGS

For the purpose of illustration rather than limitation, the present disclosure will now be described according to its preferred embodiments, particularly with reference to the accompanying drawings, in which:
Fig.1 is a schematic structural diagram of a real-time blood glucose monitoring apparatus according to an embodiment of the present disclosure;
Fig.2 is a schematic structural diagram of a sensor assembly in the real-time blood glucose monitoring apparatus according to an embodiment of the present disclosure;
Fig. 3 is a schematic structural diagram of a first lower shell of the sensor assembly in the real-time blood glucose monitoring apparatus according to an embodiment of the present disclosure;
Fig.4 is a partial enlarged diagram of a protruding column of the sensor assembly in the real-time blood glucose monitoring apparatus according to an embodiment of the present disclosure;
Fig.5 is a schematic structural diagram of an emitter assembly in the real-time blood glucose monitoring apparatus according to an embodiment of the present disclosure;
Fig. 6 is a schematic structural diagram of a circuit board of the emitter assembly in the real-time blood glucose monitoring apparatus according to an embodiment of the present disclosure;
Fig. 7 is a structural schematic diagram of a connector of the emitter assembly in the real-time blood glucose monitoring apparatus according to an embodiment of the present disclosure;
Fig. 8 is a cross-sectional view of the connector of the emitter assembly in the real-time blood glucose monitoring apparatus according to an embodiment of the present disclosure;
Fig. 9 is a cross-sectional view of the real-time blood glucose monitoring apparatus according to an embodiment of the present disclosure;
Fig. 10 is another schematic structural diagram of the sensor assembly in the real-time blood glucose monitoring apparatus according to an embodiment of the present disclosure;
Fig. 11 is another schematic structural diagram of the emitter assembly in the real-time blood glucose monitoring apparatus according to an embodiment of the present disclosure;
Fig.12 is a flow block diagram of a manufacturing method of the real-time blood glucose monitoring apparatus according to an embodiment of the present disclosure.

In the drawings:
1: sensor assembly; 2: emitter assembly;
11: first shell; 12: conductive plating; 13: first connecting structure; 14: first clamping structure; 111: first upper shell; 112: first lower shell; 121: to-be-connected terminals; 131: protruding column; 1111: first sleeve; 1112: first annular insertion groove; 1113: sealing ring;
21: circuit board; 22: second shell; 23: second connecting structure; 24: second clamping structure; 221: second upper shell; 222: second lower shell; 231: connector; 232: plug-in recess; 233: reed; 2221: second sleeve; 2222: second annular insertion groove.

### DETAILED DESCRIPTION OF EXAMPLES

In the embodiments of the present disclosure, by optimizing the structures of internal elements of a blood glucose monitoring apparatus, the volume of products can be reduced, which further facilitates the miniaturization of the products, as described in detail below.

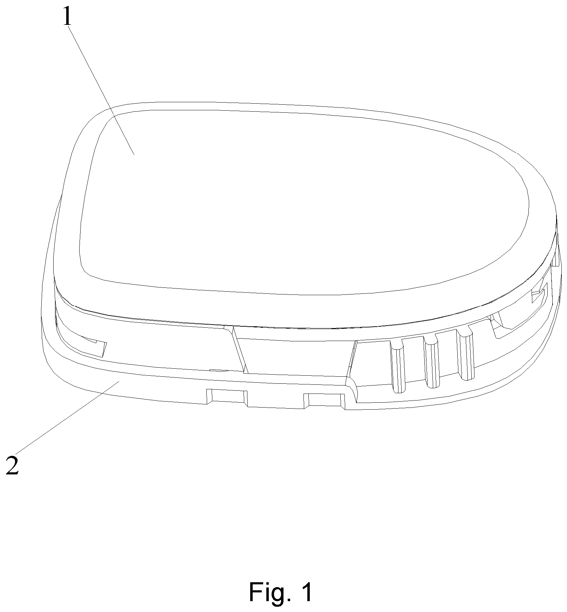

Fig. 1 is a schematic structural diagram of a real-time blood glucose monitoring apparatus according to an embodiment of the present disclosure. As shown in Fig.1, the real-time blood glucose monitoring apparatus comprises a sensor assembly 1 and an emitter assembly 2, both of which are split structures. When in use, the sensor assembly 1 is used to collect blood glucose value data, which is for one-time use. The emitter assembly 2 comprises a circuit board 21. The emitter assembly 2 can be connected to the sensor assembly 1 to form a closed-loop monitoring circuit. The blood glucose value data collected by the sensor assembly 1 is sent to a terminal device via the emitter assembly 2. Since the emitter assembly 2 is not in direct contact with human skin, it can be reused. In the blood glucose monitoring apparatus, the sensor assembly 1 needs to be used for a period of time, and after the use cycle is over, the power of a battery is basically exhausted. Therefore, the battery can be arranged in the sensor assembly 1 for one-time use.

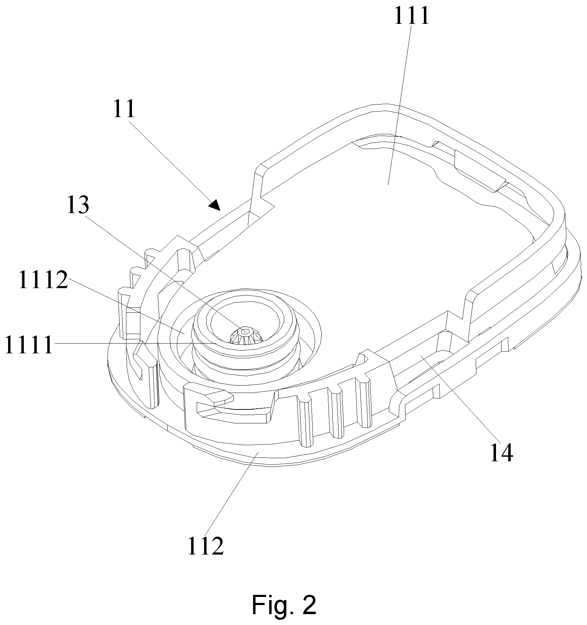

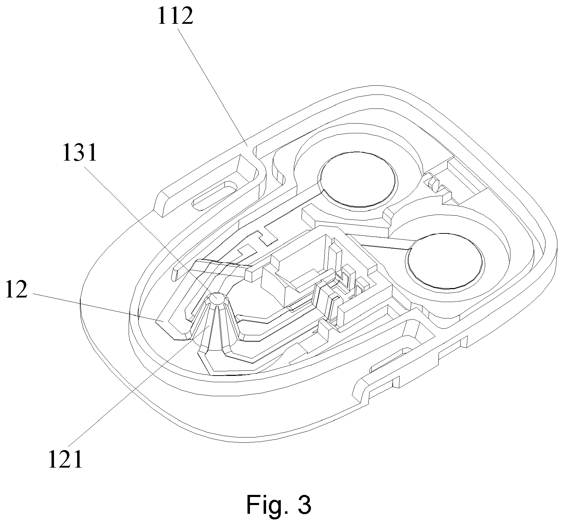

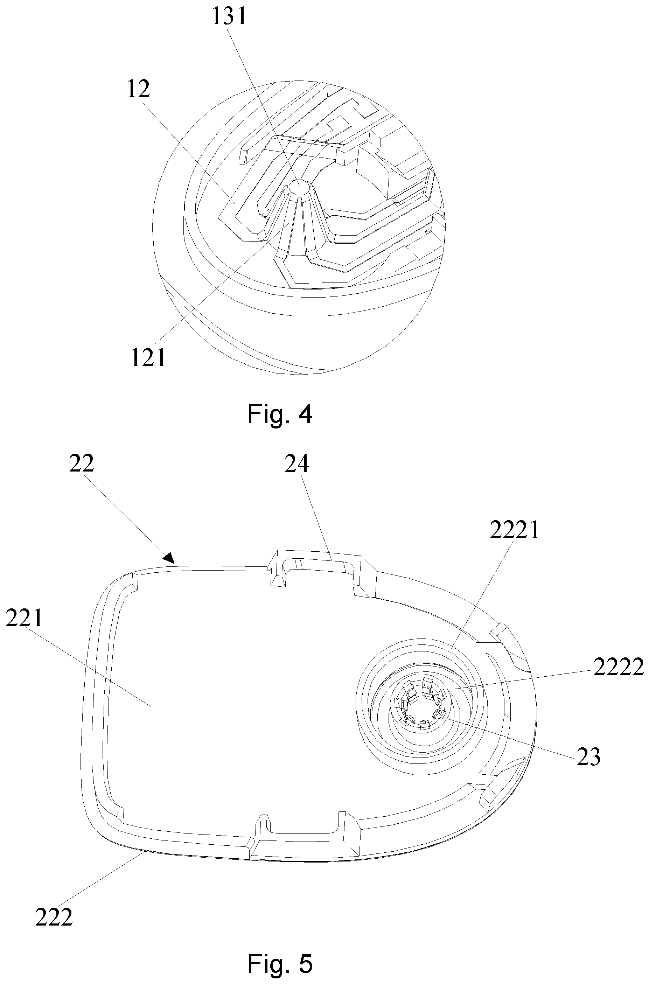

Fig.2 is a schematic structural diagram of the sensor assembly in the real-time blood glucose monitoring apparatus according to an embodiment of the present disclosure; Fig. 3 is a schematic structural diagram of a first lower shell of the sensor assembly in the real-time blood glucose monitoring apparatus according to an embodiment of the present disclosure; and Fig.4 is a partial enlarged diagram of a protruding column of the sensor assembly in the real-time blood glucose monitoring apparatus according to an embodiment of the present disclosure. As shown in Figs. 2 to 4, the sensor assembly 1 comprises a first shell 11. The first shell 11 is provided with a conductive plating 12 connected to the first shell 11. The blood glucose sensor and the battery are both arranged in the first shell 11 and connected to the conductive plating 12.The conductive plating 12 has at least one first connecting structure 13 protruding from the first shell 11. The conductive plating 12 replaces a circuit board structure in the sensor assembly 1, and thus the space required for installing the circuit board structure in the first shell 11 can be saved, thereby reducing the volume of the sensor assembly 1. The conductive plating 12 includes a plurality of metal wires, which are plated on the first shell 11. Preferably, the metal wires are plated on the first shell 11 using a molded interconnect device (MID) process or a laser direct structuring (LDS) process. In a preferred example of an embodiment of the present disclosure, as shown in Figs. 2 and 3, the first shell 11 comprises a first upper shell 111 and a first lower shell 112, the edges of which are connected. The conductive plating 12 is arranged on an inner surface of the first lower shell 112, and the protruding column 131 is fixedly connected to the inner surface of the first lower shell 112.

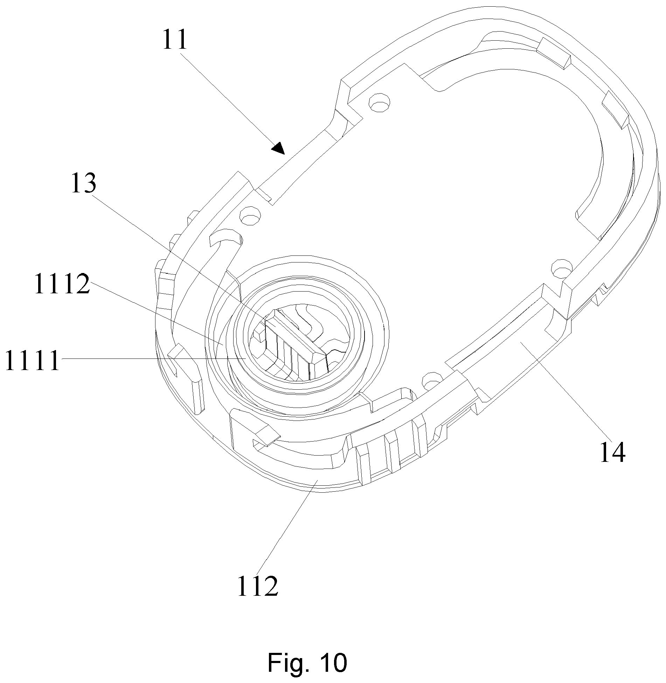

Continuing to refer to Figs. 2 to4, as shown in the figures, the first connecting structure 13 is a protruding column 131, which is arranged in the first shell 11, and a first end of which penetrates the first shell 11 to be located outside the first shell 11. A plurality of to-be-connected terminals 121 of the conductive plating 12 are arranged on an outer wall of the protruding column 131. In an embodiment of the present disclosure, the first connecting structure 13 is used as a contact for connection with the emitter assembly 2. Due to the use of the conductive plating 12 in the sensor assembly 1, when the first connecting structure 13 is provided, the to-be-connected terminals 121 of the metal wires in the conductive plating 12 can be are centrally arranged on the protruding column 131, thereby decreasing the number of contacts and achieving the purpose of further reducing the volume of the sensor assembly 1. Preferably, in the sensor assembly 1, only one first connecting structure 13 is provided. By distributing the plurality of to-be-connected terminals 121 of the conductive plating 12 along the outer wall of the protruding column 131, the to-be-connected terminals 121 can be prevented from being affected by each other. In an embodiment of the present disclosure, the to-be-connected terminals 121 can be arranged in various ways on the first connecting structure 13. As shown in Fig. 4, the protruding column 131 is conical form, and the plurality of to-be-connected terminals 121 are distributed circumferentially on the conical structure. Fig. 10 is another schematic structural diagram of the sensor assembly in the real-time blood glucose monitoring apparatus according to an embodiment of the present disclosure. As shown in Fig. 10, the protruding column 131 is in the shape of a cuboid, and the plurality of to-be-connected terminals 121 are arranged on two opposite surfaces of the protruding column 131.

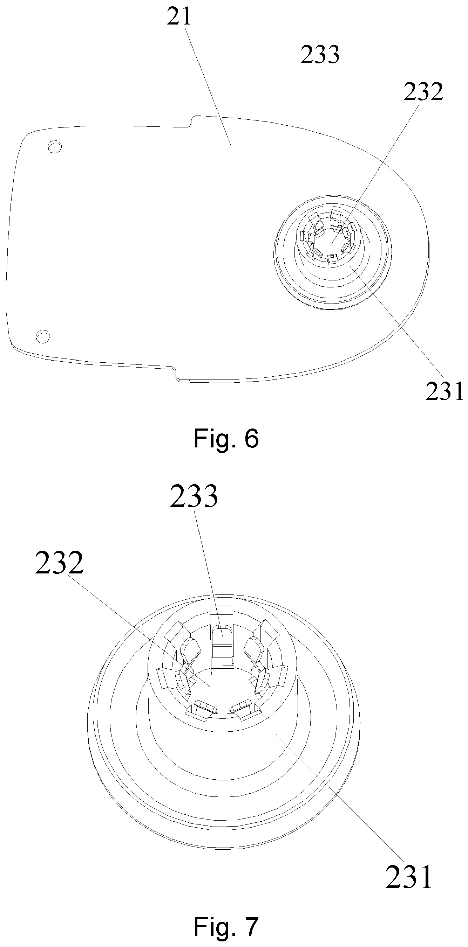

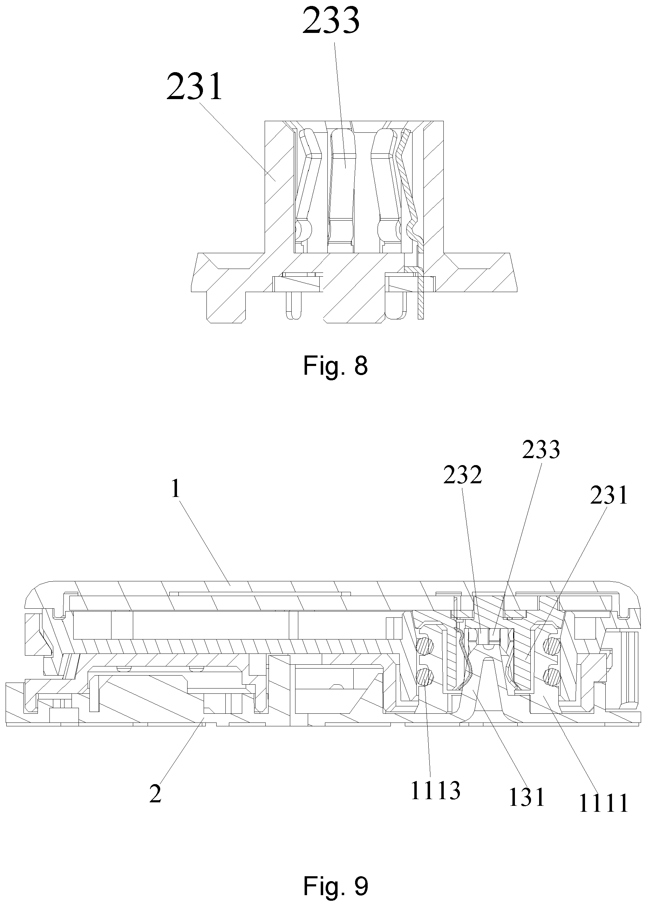

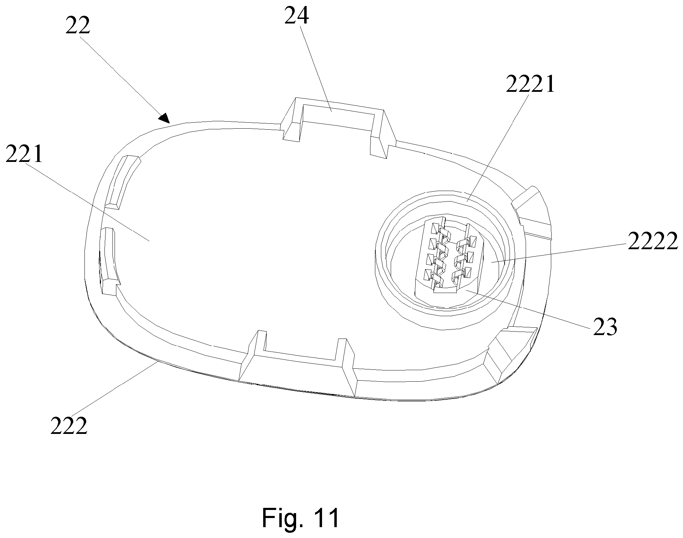

Fig.5 is a schematic structural diagram of the emitter assembly in the real-time blood glucose monitoring apparatus according to an embodiment of the present disclosure; Fig. 6 is a schematic structural diagram of a circuit board of the emitter assembly in the real-time blood glucose monitoring apparatus according to an embodiment of the present disclosure; Fig. 7 is a structural schematic diagram of a connector of the emitter assembly in the real-time blood glucose monitoring apparatus according to an embodiment of the present disclosure; and Fig. 8 is a cross-sectional view of the connector of the emitter assembly in the real-time blood glucose monitoring apparatus according to an embodiment of the present disclosure. As shown in Figs. 5 to 8, the emitter assembly 2 comprises a second shell 22. The second shell 22 comprises a circuit board 21, which is provided with at least one second connecting structure 23 protruding from the second shell 22. The second connecting structure 23 is a connector 231. There is no specific limitation on the shape of the connector 231 in the embodiment of the present disclosure. Thus, the connector 231 may be of any shape in the event of being able to ensure connection to the protruding column 131, for example, as shown in Figs. 5 to 8, the connector 231 is a circular connector, which can be adapted to the protruding column131 shown in Figs. 2 to 4. Fig. 11 is another schematic structural diagram of the emitter assembly in the real-time blood glucose monitoring apparatus according to an embodiment of the present disclosure. As shown in Fig. 11, the connector 231 has a shape approximating to a cuboid, in a middle portion of which is provided a square-shaped plug-in recess 232 that can be adapted to the protruding column 131 shown in Fig.10. The first end of the connector 231 is connected to the circuit board 21. The second end of the connector 231 penetrates the second shell 11 to be located outside the second shell 22, and is provided with an inwardly concave plug-in recess 232.On an inner wall of the plug-in recess 232 are provided reeds 233 connected to the circuit board 21. The second connecting structure 23 is adapted to the first connecting structure 13, and the number of the second connecting structure 23 is the same as that of the first connecting structure 13. When the second connecting structure 23 and the first connecting structure 13 are connected, the protruding column 131 is plugged into the plug-in recess 232, and the to-be-connected terminals 121 on the outer wall of the protruding column 131 are connected to the reeds 233, so that the elements such as the battery and the blood glucose sensor in the sensor assembly 1 communicate with the circuit board 21, thereby making it possible to form a closed-loop monitoring circuit.

Fig. 9 is a cross-sectional view of the real-time blood glucose monitoring apparatus according to an embodiment of the present disclosure. As shown in Figs. 1 to 9, the second shell 22 comprises a second upper shell 221 and a second lower shell 222, the edges of which are connected. A clamping structure is provided on an edge of the outer surface of the first upper shell 111 and an edge of the outer surface of the second lower shell 222 respectively. The clamping structures are the first clamping structure 14 and the second clamping structure 24. The outer surface of the first upper shell 111 and the outer surface of the second lower shell 222 are put together so that the clamping structures are clamped. The first shell 11 and the second shell 22 are clamped so that the first connecting structure 13 is plugged into and connected with the second connecting structure 23. In the embodiment of the present disclosure, the first connecting structure 13 and the second connecting structure 23 are located on the asymmetrical axis of the sensor assembly 1 and the emitter assembly 2. The outer surface of the first lower shell 112 is provided with medical adhesive tape. The blood glucose sensor is arranged in the middle portion of the inner surface of the lower shell 112, and a detection end of the blood glucose sensor penetrates the first lower shell 112 and the medical adhesive tape to be located outside the first shell 11. By adopting the setting of this position, the layout of the elements in the sensor assembly 1 can be optimized, and the sensor assembly 1 and the emitter assembly 2 can be prevented from being installed in the wrong direction, thereby facilitating the rapid installation of both.

As shown in Figs. 2, 5 and 9, the first upper shell 111 is provided with a first through-hole, an edge of which is provided with a first sleeve 1111 protruding from the outer surface of the first upper shell 111.The protruding column131is arranged within and runs through the first sleeve 1111. A first annular insertion groove 1112 is formed between an outer wall of the first sleeve 1111 and the first upper shell 111. The center lines of the protruding column 131, the first sleeve 1111 and the second annular insertion groove 2222 coincide. The second lower shell 222 is provided with a second through-hole, an edge of which is provided with a second sleeve 2221 protruding from an outer surface of the second lower shell 222. The connector 231 is arranged within and runs through the second sleeve 2221, and there is a defined spacing between the outer wall of the connector 231 and the inner wall of the second sleeve 2221, thereby forming a second annular insertion groove 2222. The center lines of the connector 231, the second sleeve 2221 and the second annular insertion groove 2222 coincide. When the first connecting structure 13 and the second connecting structure 23 are connected, the first sleeve 1111 is inserted into the second annular insertion groove 2222, and the second sleeve 2221 is inserted into the first annular insertion groove 1112. By setting the structures of the first sleeve 1111 and the second sleeve 2221, the connection between the first connecting structure 13 and the second connecting structure 23is more tightening and stable, and has a good sealing effect, when the first connecting structure 13is plugged into the second connecting structure 23. Preferably, at least one sealing ring 1113 is sleeved on the outer wall of the first sleeve 1111. The sealing ring 1113 blocks a gap between the first sleeve 1111 and the second sleeve 2221 when the first connecting structure 13 and the second connecting structure 23 are connected. By using the sealing ring 1113, there is a better sealing effect between the first sleeve 1111 and the second sleeve 2221.

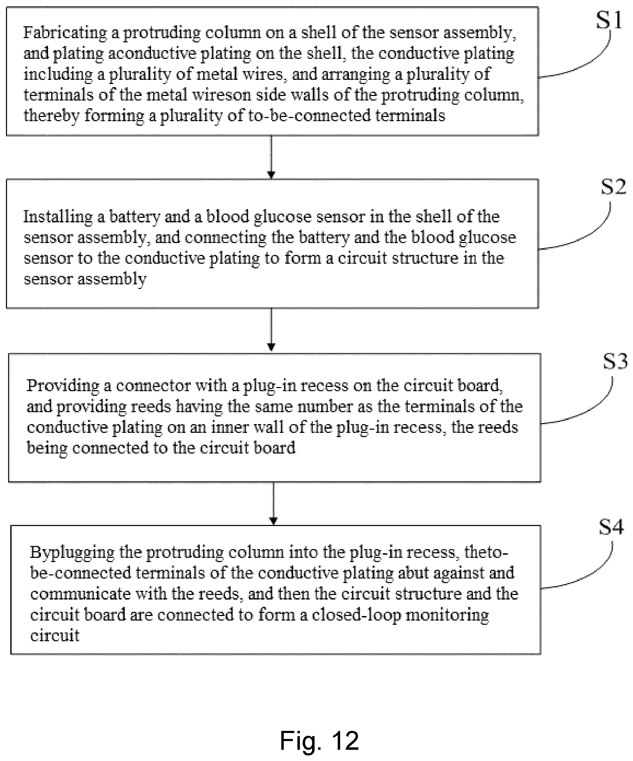

Fig.12 is a flow block diagram of a manufacturing method of the real-time blood glucose monitoring apparatus according to an embodiment of the present disclosure. As shown in Fig.12, the manufacturing process of the real-time blood glucose monitoring apparatus comprises the following steps:
Step S1: incorporating a protruding column onto the shell of the sensor assembly, for example, molding the protruding column and the shell of the sensor assembly in one piece, or fabricating the protruding column on the inner wall of the shell of the sensor assembly; and plating a conductive plating on the shell, the conductive plating including a plurality of metal wires, and arranging a plurality of terminals of the metal wires on sidewalls of the protruding column, thereby forming a plurality of to-be-connected terminals; preferably, the conductive plating is plated on the shell of the sensor assembly by molded interconnection device (MID)process and/or laser direct forming (LDS) process.
Step S2: installing a battery and a blood glucose sensor in the shell of the sensor assembly, and connecting the battery and the blood glucose sensor to the conductive plating to form a circuit structure in the sensor assembly;
Step S3: providing a connector including a plug-in recess on the circuit board, and providing reeds having the same number as the terminals of the conductive plating on an inner wall of the plug-in recess, the reeds being connected to the circuit board; Step S4: by plugging the protruding column into the plug-in recess, the to-be-connected terminals of the conductive plating abut against and communicate with the reeds, and then the circuit structure and the circuit board are connected to form a closed-loop monitoring circuit.

According to the technical solution of the embodiment of the present disclosure, a conductive plating is provided on the shell of the sensor assembly. The conductive plating replaces the existing circuit board structures. By reducing circuit board structures, the sensor assembly can be further miniaturized. Furthermore, by means of the conductive plating, contacts for connection can be arranged in a centralized manner, such that distribution points of the contacts for connection on the sensor assembly and the emitter assembly can be reduced, and the space occupied by a contact structure can be reduced, thereby facilitating the miniaturization of the product. The above specific embodiments do not constitute a limitation on the scope of protection of the present invention; the scope of which is defined by the appended claims. It should be apparent to those skilled in the art that various modifications, combinations, sub-combinations and substitutions may take place depending on design requirements and other factors.

## Claims

1. A real-time blood glucose monitoring apparatus, comprising a sensor assembly (1) and an emitter assembly (2), wherein
the sensor assembly (1) comprises a first shell (11), and a conductive plating (12), a blood glucose sensor and a battery which are arranged on the first shell(11), and the emitter assembly (2) comprises a second shell (22) and a circuit board (21) in the second shell (22), the first shell (11) comprises a first upper shell (111) and a first lower shell (112), the edges of which are connected, the second shell (22) comprises a second upper shell (221) and a second lower shell (222), the edges of which are connected;
the conductive plating (12) is arranged on a shell wall of the first shell (11) and is connected with the blood glucose sensor and the battery, the conductive plating (12) having at least one first connecting structure (13), the first connecting structure (13) is a protruding column (131), the conductive plating (12) is arranged on a surface of the first lower shell (112), and the protruding column (131) is fixedly connected to the surface of the first lower shell (112);
the circuit board (21) is provided with at least one second connecting structure (23), the second connecting structure (23) is a connector (231);
the sensor assembly (1) and the emitter assembly (2) are clamped so that the first connecting structure (13) and the second connecting structure (23) are connected, and the conductive plating (12) and the circuit board (21) form a closed-loop monitoring circuit, **characterised in that**
the first upper shell (111) is provided with a first through-hole, an edge of which is provided with a first sleeve (1111); the protruding column (131) is arranged within and runs through the first sleeve (1111); a first annular insertion groove (1112) is formed between an outer wall of the first sleeve (1111) and the first upper shell (111); and the center lines of the protruding column (131), the first sleeve (1111) and the first annular insertion groove (1112) coincide;
the second lower shell (222) is provided with a second through-hole, an edge of which is provided with a second sleeve (2221) protruding from an outer surface of the second lower shell (222); the connector (231) is arranged within and runs through the second sleeve (2221), and there is a defined spacing between an outer wall of the connector (231) and an inner wall of the second sleeve (2221), thereby forming a second annular insertion groove (2222); the center lines of the connector (231), the second sleeve (2221) and the second annular insertion groove (2222) coincide;
when the first connecting structure (13) and the second connecting structure (23) are connected, the first sleeve (1111) is inserted into the second annular insertion groove (2222), and the second sleeve (2221) is inserted into the first annular insertion groove (1112).

2. The real-time blood glucose monitoring apparatus according to claim 1, **characterized in that** a plurality of to-be-connected terminals (121) of the conductive plating (12) are provided on a wall of the protruding column (131);
a first end of the connector (231) is connected with the circuit board (21) and a second end of which is provided with an inwardly-concave plug-in recess (232), and reeds (233) connected to the circuit board (21) are provided on an inner wall of the plug-in recess (232);
when the first connecting structure (13) and the second connecting structure (23) are connected, the protruding column (131) is plugged into the plug-in recess (232), and the reeds (233) abut against the to-be-connected terminals (121) of the conductive plating (12).

3. The real-time blood glucose monitoring apparatus according to claim 2, **characterized in that** the plurality of to-be-connected terminals (121) of the conductive plating (12) are distributed along the wall of the protruding column (131).

4. The real-time blood glucose monitoring apparatus according to claim 1, **characterized in that** a sealing structure for blocking a gap between the first sleeve (1111) and the second sleeve (2221) is provided between the outer wall of the first sleeve (1111) and the inner wall of the second sleeve (2221).

5. The real-time blood glucose monitoring apparatus according to claim 4, **characterized in that** the sealing structure comprises at least one sealing ring (1113), which is sleeved on the outer wall of the first sleeve (1111).

6. The real-time blood glucose monitoring apparatus according to claim 1, **characterized in that** an outer surface of the first lower shell (112) is provided with medical adhesive tape, and the blood glucose sensor is arranged in a middle portion of an inner surface of the first lower shell (112), with a detecting end of the blood glucose sensor penetrating out of the first lower shell (112).

7. A method for manufacturing a real-time blood glucose monitoring apparatus according to any one of claims 1 to 6, the method **characterized by** comprising:
plating conductive plating on a shell of a sensor assembly, and connecting a battery and a blood glucose sensor to the conductive plating to form a circuit structure in the sensor assembly;
providing a first connecting structure connected to the circuit structure, providing a second connecting structure connected to a circuit board of an emitter assembly, and communicating the first connecting structure with the second connecting structure so that the circuit structure and the circuit board are connected to form a closed-loop monitoring circuit.

8. The method according to claim 7, **characterized in that** the step of providing a first connecting structure connected to the circuit structure comprises: incorporating a protruding column onto the shell of the sensor assembly or fabricating a protruding column on the shell of the sensor assembly; arranging a plurality of to-be-connected terminals of the conductive plating on side walls of the protruding column;
the step of providing a second connecting structure connected with a circuit board of an emitter assembly comprises: providing a connector with a plug-in recess on the circuit board, and providing reeds having the same number as the terminals of the conductive plating on an inner wall of the plug-in recess, the reeds being connected to the circuit board;
by plugging the protruding column into the plug-in recess, the to-be-connected terminals of the conductive plating abut against and communicates with the reeds, and then the circuit structure and the circuit board are connected to form a closed-loop monitoring circuit.

9. The method according to claim 7, **characterized in that** the conductive plating is plated on the shell of the sensor assembly by molded interconnection device process and/or laser direct structuring process.

## Patentansprüche

1. Eine Echtzeit-Blutglukoseüberwachungsvorrichtung, umfassend eine Sensoranordnung (1) und eine Emitteranordnung (2), wobei
die Sensoranordnung (1) eine erste Schale (11) und eine leitfähige Beschichtung (12), einen Blutglukosesensor und eine Batterie umfasst, die auf der ersten Schale (11) angeordnet sind, und die Emitteranordnung (2) eine zweite Schale (22) und eine Leiterplatte (21) in der zweiten Schale (22) umfasst, die erste Schale (11) eine erste obere Schale (111) und eine erste untere Schale (112) umfasst, deren Ränder verbunden sind, die zweite Schale (22) eine zweite obere Schale (221) und eine zweite untere Schale (222) umfasst, deren Ränder verbunden sind;
die leitfähige Beschichtung (12) an einer Schalenwand der ersten Schale (11) angeordnet und mit dem Blutglukosesensor und der Batterie verbunden ist, wobei die leitfähige Beschichtung (12) mindestens eine erste Verbindungsstruktur (13) aufweist, die erste Verbindungsstruktur (13) eine vorstehende Säule (131) ist, die leitfähige Beschichtung (12) an einer Oberfläche der ersten unteren Schale (112) angeordnet ist und die vorstehende Säule (131) fest mit der Oberfläche der ersten unteren Schale (112) verbunden ist;
die Leiterplatte (21) mit mindestens einer zweiten Verbindungsstruktur (23) versehen ist und die zweite Verbindungsstruktur (23) ein Verbinder (231) ist;
die Sensoranordnung (1) und die Emitteranordnung (2) so geklemmt sind, dass die erste Verbindungsstruktur (13) und die zweite Verbindungsstruktur (23) verbunden sind und die leitfähige Beschichtung (12) und die Leiterplatte (21) einen geschlossenen Überwachungskreis bilden, **dadurch gekennzeichnet, dass**
die erste obere Schale (111) mit einem ersten Durchgangsloch versehen ist, von dem ein Rand mit einer ersten Hülse (1111) versehen ist; die vorstehende Säule (131) innerhalb der ersten Hülse (1111) angeordnet ist und durch diese verläuft; eine erste ringförmige Einführungsnut (1112) zwischen einer Außenwand der ersten Hülse (1111) und der ersten oberen Schale (111) gebildet ist; und die Mittellinien der vorstehenden Säule (131), der ersten Hülse (1111) und der ersten ringförmigen Einführungsnut (1112) zusammenfallen;
die zweite untere Schale (222) mit einem zweiten Durchgangsloch versehen ist, von dem ein Rand mit einer zweiten Hülse (2221) versehen ist, die von einer Außenfläche der zweiten unteren Schale (222) vorsteht; der Verbinder (231) innerhalb der zweiten Hülse (2221) angeordnet ist und durch diese verläuft und ein definierter Abstand zwischen einer Außenwand des Verbinders (231) und einer Innenwand der zweiten Hülse (2221) besteht, wodurch eine zweite ringförmige Einführungsnut (2222) gebildet wird; die Mittellinien des Verbinders (231), der zweiten Hülse (2221) und der zweiten ringförmigen Einführungsnut (2222) zusammenfallen;
wenn die erste Verbindungsstruktur (13) und die zweite Verbindungsstruktur (23) verbunden sind, die erste Hülse (1111) in die zweite ringförmige Einführungsnut (2222) eingeführt wird, und die zweite Hülse (2221) in die erste ringförmige Einführungsnut (1112) eingeführt wird.

2. Die Echtzeit-Blutglukoseüberwachungsvorrichtung nach Anspruch 1, **dadurch gekennzeichnet, dass** eine Vielzahl von zu verbindenden Anschlüssen (121) der leitfähigen Beschichtung (12) an einer Wand der vorstehenden Säule (131) bereitgestellt ist;
ein erstes Ende des Verbinders (231) mit der Leiterplatte (21) verbunden ist und ein zweites Ende davon mit einer nach innen konkaven Steckausnehmung (232) versehen ist, und Zungen (233), die mit der Leiterplatte (21) verbunden sind, an einer Innenwand der Steckausnehmung (232) vorgesehen sind;
wenn die erste Verbindungsstruktur (13) und die zweite Verbindungsstruktur (23) verbunden sind, die vorstehende Säule (131) in die Steckausnehmung (232) gesteckt wird, und die Zungen (233) gegen die zu verbindenden Anschlüsse (121) der leitfähigen Beschichtung (12) stoßen.

3. Die Echtzeit-Blutglukoseüberwachungsvorrichtung nach Anspruch 2, **dadurch gekennzeichnet, dass** die Vielzahl von zu verbindenden Anschlüssen (121) der leitfähigen Beschichtung (12) entlang der Wand der vorstehenden Säule (131) verteilt sind.

4. Die Echtzeit-Blutglukoseüberwachungsvorrichtung nach Anspruch 1, **dadurch gekennzeichnet, dass** eine Dichtungsstruktur zum Blockieren eines Spalts zwischen der ersten Hülse (1111) und der zweiten Hülse (2221) zwischen der Außenwand der ersten Hülse (1111) und der Innenwand der zweiten Hülse (2221) vorgesehen ist.

5. Das Echtzeit-Blutglukoseüberwachungsvorrichtung nach Anspruch 4, **dadurch gekennzeichnet, dass** die Dichtungsstruktur mindestens einen Dichtungsring (1113) umfasst, der an der Außenwand der ersten Hülse (1111) ummantelt ist.

6. Die Echtzeit-Blutglukoseüberwachungsvorrichtung nach Anspruch 1, **dadurch gekennzeichnet, dass** eine Außenfläche der ersten unteren Schale (112) mit einem medizinischen Klebeband versehen ist und der Blutglukosesensor in einem mittleren Abschnitt einer Innenfläche der ersten unteren Schale (112) angeordnet ist, wobei ein Erfassungsende des Blutglukosesensors aus der ersten unteren Schale (112) herausdringt.

7. Ein Verfahren zur Herstellung einer Echtzeit-Blutglukoseüberwachungsvorrichtung nach einem der Ansprüche 1 bis 6, wobei das Verfahren **dadurch gekennzeichnet ist, dass** es Folgendes umfasst:
aufbringen einer leitfähigen Beschichtung auf einer Hülle einer Sensoranordnung und Verbinden einer Batterie und eines Blutglukosesensors mit der leitfähigen Beschichtung, um eine Schaltungsstruktur in der Sensoranordnung zu bilden;
bereitstellen einer ersten Verbindungsstruktur, die mit der Schaltungsstruktur verbunden ist, bereitstellen einer zweiten Verbindungsstruktur, die mit einer Leiterplatte einer Emitteranordnung verbunden ist, und kommunizieren der ersten Verbindungsstruktur mit der zweiten Verbindungsstruktur, so dass die Schaltungsstruktur und die Leiterplatte verbunden sind, um einen geschlossenen Überwachungskreis zu bilden.

8. Das Verfahren nach Anspruch 7, **dadurch gekennzeichnet, dass** der Schritt des Bereitstellens einer ersten Verbindungsstruktur, die mit der Schaltungsstruktur verbunden ist, Folgendes umfasst: einbauen einer vorstehenden Säule auf die Hülle der Sensoranordnung oder herstellen einer vorstehenden Säule auf der Hülle der Sensoranordnung; anordnen einer Vielzahl von zu verbindenden Anschlüssen der leitfähigen Beschichtung an Seitenwänden der vorstehenden Säule;
der Schritt des Bereitstellens einer zweiten Verbindungsstruktur, die mit einer Leiterplatte einer Emitteranordnung verbunden ist, Folgendes umfasst: bereitstellen eines Verbinders mit einer Steckausnehmung auf der Leiterplatte und bereitstellen von Zungen mit der gleichen Anzahl wie die Anschlüsse der leitfähigen Beschichtung an einer Innenwand der Steckausnehmung, wobei die Zungen mit der Leiterplatte verbunden sind;
durch Einstecken der vorstehenden Säule in die Steckausnehmung stoßen die zu verbindenden Anschlüsse der leitfähigen Beschichtung gegen die Zungen und stehen mit diesen in Verbindung, und dann werden die Schaltungsstruktur und die Leiterplatte verbunden, um einen geschlossenen Überwachungskreis zu bilden.

9. Das Verfahren nach Anspruch 7, **dadurch gekennzeichnet, dass** die leitfähige Beschichtung auf der Hülle der Sensoranordnung durch einen Prozess geformter Verbindungsvorrichtung und/oder einen Laserdirektstrukturierungsprozess aufgebracht wird.

## Revendications

1. Un appareil de surveillance de la glycémie en temps réel, comprenant un ensemble capteur (1) et un ensemble émetteur (2), dans lequel
l'ensemble capteur (1) comprend une première coque (11), et un revêtement conducteur (12), un capteur de glycémie et une batterie qui sont agencés sur la première coque (11), et l'ensemble émetteur (2) comprend une seconde coque (22) et une carte électronique (21) dans la seconde coque (22), la première coque (11) comprend une première coque supérieure (111) et une première coque inférieure (112), dont les bords sont connectés, la seconde coque (22) comprend une seconde coque supérieure (221) et une seconde coque inférieure (222), dont les bords sont connectés ;
le revêtement conducteur (12) est disposé sur une paroi de coque de la première coque (11) et est connecté au capteur de glycémie et à la batterie, le revêtement conducteur (12) ayant au moins une première structure de connexion (13), la première structure de connexion (13) est une colonne en saillie (131), le revêtement conducteur (12) est disposé sur une surface de la première coque inférieure (112), et la colonne en saillie (131) est connectée de manière fixe à la surface de la première coque inférieure (112) ;
la carte électronique (21) est pourvue d'au moins une seconde structure de connexion (23), la seconde structure de connexion (23) est un connecteur (231) ;
l'ensemble capteur (1) et l'ensemble émetteur (2) sont serrés de sorte que la première structure de connexion (13) et la seconde structure de connexion (23) sont connectées, et le revêtement conducteur (12) et la carte électronique (21) forment un circuit de surveillance en boucle fermée, **caractérisé en ce que**
la première coque supérieure (111) est pourvue d'un premier trou traversant, dont un bord est pourvu d'un premier manchon (1111) ; la colonne en saillie (131) est agencée à l'intérieur et traverse le premier manchon (1111) ; une première rainure d'insertion annulaire (1112) est formée entre une paroi extérieure du premier manchon (1111) et la première coque supérieure (111) ; et les lignes centrales de la colonne en saillie (131), du premier manchon (1111) et de la première rainure d'insertion annulaire (1112) coïncident ;
la seconde coque inférieure (222) est pourvue d'un second trou traversant, dont un bord est pourvu d'un second manchon (2221) faisant saillie d'une surface extérieure de la seconde coque inférieure (222) ; le connecteur (231) est agencé à l'intérieur de et traverse le second manchon (2221), et il y a un espacement défini entre une paroi extérieure du connecteur (231) et une paroi intérieure du second manchon (2221), formant ainsi une seconde rainure d'insertion annulaire (2222) ; les lignes centrales du connecteur (231), du second manchon (2221) et de la seconde rainure d'insertion annulaire (2222) coïncident ;
lorsque la première structure de connexion (13) et la seconde structure de connexion (23) sont connectées, le premier manchon (1111) est inséré dans la seconde rainure d'insertion annulaire (2222), et le second manchon (2221) est inséré dans la première rainure d'insertion annulaire (1112).

2. L'appareil de surveillance de la glycémie en temps réel selon la revendication 1, **caractérisé en ce qu'**une pluralité de bornes à connecter (121) du revêtement conducteur (12) sont prévues sur une paroi de la colonne en saillie (131) ;
une première extrémité du connecteur (231) est connectée à la carte électronique (21) et dont une seconde extrémité est pourvue d'un évidement d'enfichage concave vers l'intérieur (232), et des lames (233) connectées à la carte électronique (21) sont prévues sur une paroi intérieure de l'évidement d'enfichage (232) ;
lorsque la première structure de connexion (13) et la seconde structure de connexion (23) sont connectées, la colonne en saillie (131) est enfichée dans l'évidement d'enfichage (232), et les lames (233) viennent en butée contre les bornes à connecter (121) du revêtement conducteur (12).

3. L'appareil de surveillance de la glycémie en temps réel selon la revendication 2, **caractérisé en ce que** la pluralité de bornes à connecter (121) du revêtement conducteur (12) sont distribuées le long de la paroi de la colonne en saillie (131).

4. L'appareil de surveillance de la glycémie en temps réel selon la revendication 1, **caractérisé en ce qu'**une structure d'étanchéité pour bloquer un espace entre le premier manchon (1111) et le second manchon (2221) est prévue entre la paroi externe du premier manchon (1111) et la paroi interne du second manchon (2221).

5. L'appareil de surveillance de la glycémie en temps réel selon la revendication 4, **caractérisé en ce que** la structure d'étanchéité comprend au moins un anneau d'étanchéité (1113), qui est manchonné sur la paroi extérieure du premier manchon (1111).

6. L'appareil de surveillance de la glycémie en temps réel selon la revendication 1, **caractérisé en ce qu'**une surface extérieure de la première coque inférieure (112) est pourvue d'un ruban adhésif médical, et le capteur de glycémie est agencé dans une partie médiane d'une surface intérieure de la première coque inférieure (112), avec une extrémité de détection du capteur de glycémie pénétrant hors de la première coque inférieure (112).

7. Un procédé pour fabriquer un appareil de surveillance de la glycémie en temps réel selon l'une quelconque des revendications 1 à 6, le procédé étant **caractérisé en ce qu'**il comprend :
plaquer un revêtement conducteur sur une coque d'un ensemble capteur, et connecter une batterie et un capteur de glycémie au revêtement conducteur pour former une structure de circuit dans l'ensemble capteur ;
fournir une première structure de connexion connectée à la structure de circuit, fournir une seconde structure de connexion connectée à une carte de circuit d'un ensemble émetteur, et communiquer la première structure de connexion avec la seconde structure de connexion de sorte que la structure de circuit et la carte électronique soient connectées pour former un circuit de surveillance en boucle fermée.

8. Le procédé selon la revendication 7, **caractérisé en ce que** l'étape consistant à fournir une première structure de connexion connectée à la structure de circuit comprend : incorporer une colonne en saillie sur la coque de l'ensemble de capteurs ou fabriquer une colonne en saillie sur la coque de l'ensemble de capteurs ; agencer une pluralité de bornes à connecter du revêtement conducteur sur des parois latérales de la colonne en saillie ;
l'étape de fournir une seconde structure de connexion connectée à une carte électronique d'un ensemble émetteur comprend : fournir un connecteur avec un évidement d'enfichage sur la carte électronique, et fournir des lames ayant le même nombre que les bornes du revêtement conducteur sur une paroi intérieure de l'évidement d'enfichage, les lames étant connectées à la carte électronique ;
en enfichant la colonne en saillie dans l'évidement d'enfichage, les bornes à connecter du revêtement conducteur butent contre et communiquent avec les lames, puis la structure de circuit et la carte électronique sont connectées pour former un circuit de surveillance en boucle fermée.

9. Le procédé selon la revendication 7, **caractérisé en ce que** le revêtement conducteur est plaqué sur la coque de l'ensemble capteur par un processus de dispositif d'interconnexion moulé et/ou un processus de structuration directe par laser.
